# EUROPEAN PATENT APPLICATION

(11) **EP 4 084 014 A1**
(43) Date of publication of application: **02.11.2022**
(21) Application number: 20904309.0
(22) Date of filing: 22.12.2020
(51) Int. Cl.: G16H 50/30, G06T 7/00, G06T 7/246

(54) **CHEWING SUPPORT SYSTEM**

(30) Priority: 26.12.2019 JP 2019237304
(71) Applicant: SUNSTAR INC., Takatsuki-shi, Osaka 569-1195 (JP)
(72) Inventor: KANATA Takeshi, Takatsuki-shi, Osaka 569-1195 (JP)
(74) Representative: Vossius & Partner Patentanwälte Rechtsanwälte mbB
(86) International application number: PCT/JP2020/048016
(87) International publication number: WO 2021/132270

(57) **Abstract**

Provided is a chewing assistance system that can obtain chewing quality during a meal in a simple method without attaching a device such as an electromyograph to the body and causing a user to feel uncomfortable and applying a load on the user, and that can support improvement of chewing quality and health maintenance and promotion. Provided are moving image obtaining means (21) that obtains a moving image of a region including at least a mouth or a peripheral portion of the mouth in a face, analysis means (22) that analyzes a chewing action based on the moving image of the region obtained by the moving image obtaining means, quality determination means (23) that determines quality of the chewing action based on information of the chewing action analyzed by the analysis means, and extraction means (24) that extracts assistance information corresponding to the chewing quality determined by the quality determination means, from chewing information storage means (32).

## Description

### TECHNICAL FIELD

The present invention relates to a system for assisting in health maintenance and promotion in an oral cavity and a throat region in order to extend healthy life expectancy, and particularly relates to a system for assisting in and supporting improvement of quality of chewing as an "function of chewing and eating deliciously".

### BACKGROUND ART

Chewing food, swallowing behavior, salivation, and the like exert a great influence on the brain and the whole body, and exert a great influence on physical and mental health and healthy life expectancy. Health maintenance and enhancement in an oral cavity and a throat region are considered to consequently extend healthy life expectancy.

Particularly, sufficient chewing of solid meals is considered to lead to promotion of physical and mental growth, brain activation, enhancement of motor function, obesity inhibition, aging prevention, and sociality maintenance, to exhibit an effect of extending healthy life expectancy. Insufficient chewing such as the small number of chewing times in intake of a meal leads to deterioration of a chewing function of a growing child, and oral frailty of elderly people (see Non-patent Literature 1).

Furthermore, "partial chewing" in which chewing is performed always on the same side affects teeth and the jaw, the face, and the like to, for example, shorten lifetimes of teeth on one side, easily soil teeth by which chewing is not performed, apply a load onto the jaw joint, or distort the face, and also affects the whole body to result in, for example, distortion of the body or stiff shoulders or low back pain. Balance (occlusal interference) in occlusion between the left side and the right side is also considered to be associated with physical and affective stress, and affects both sympathetic nerve and parasympathetic nerve functions.

A device such as an electromyograph for counting the number of chewing times and a device for numerically indicating an occlusal force have been provided for measuring chewing quality. However, it is difficult to attach a device such as an electromyograph, in particular, to a child during a meal. Furthermore, since the electromyograph cannot obtain a motion of the jaw, a device that three-dimensionally measures a motion of the jaw by adhering a position sensor or the like has been known, but its scale is enlarged (for example, see Patent Literatures 1, 2.).

### CITATION LIST

### [PATENT LITERATURE]

[PTL 1] Japanese Unexamined Patent Application Publication No. H6-98865
[PTL 2] Japanese Unexamined Patent Application Publication No. 2019-47859

### [NON PATENT LITERATURE]

[NPL 1] Kobayashi Yoshinori, irai ronbun, Kogo/soshaku ga tsukuru kenkojumyo, Nichihotetsukaishi Ann Jpn Prosthodont, Soc3, p189-219, 2011 (Yoshinori Kobayashi, solicited article, A long Life Built by Mastication and Occlusion, Annals of Japan Prosthodontic Society, Soc3, p189-219, 2011)

### SUMMARY OF THE INVENTION

### PROBLEMS TO BE SOLVED BY THE INVENTION

Therefore, the present invention has been made in order to overcome the aforementioned circumstances, and an object of the present invention is to provide a chewing assistance system that can obtain chewing quality during a meal in a simple method without attaching a device such as an electromyograph to the body and causing a user to feel uncomfortable and applying a load on the user, and that can support improvement of chewing quality and health maintenance and promotion.

### SOLUTION TO THE PROBLEMS

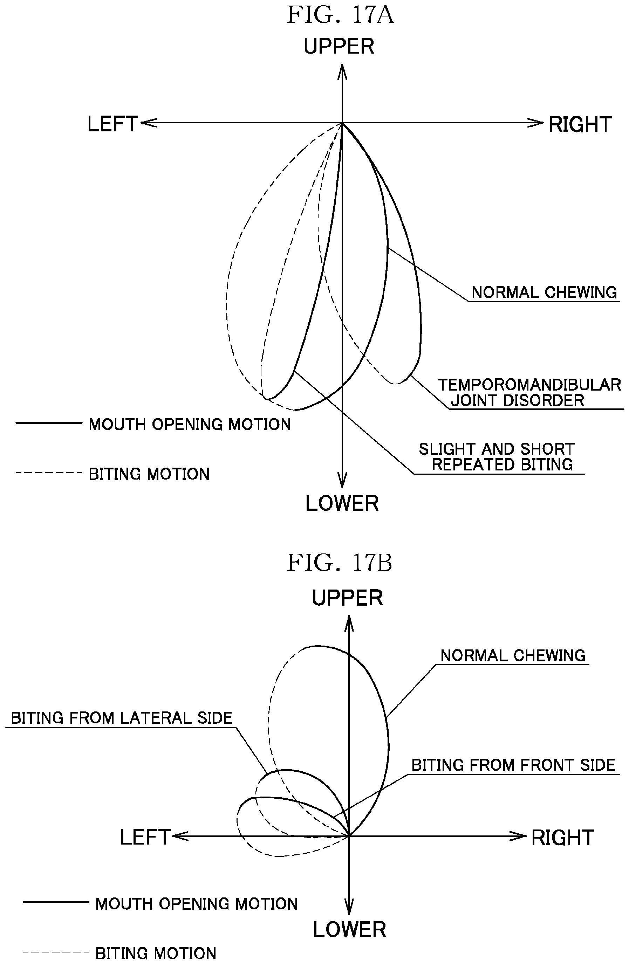

In view of the aforementioned circumstances, as a result of thorough study, the inventor of the present invention places a focus on a fact that difference in chewing behavior is found as difference in motion of a mouth or a peripheral portion of the mouth in the face. For example, a motion of the jaw in the case of chewing being performed on the right side is greatly different depending on chewing behavior as illustrated in FIG. 17. FIG. 17A illustrates the motion as viewed from the front side, and FIG. 17B illustrates the motion as horizontally viewed from thereabove.

It has been found that quality of a chewing action can be determined by analyzing an image of the motion without using a device such as an electromyograph, and assistance in enhancement of chewing quality and health maintenance and promotion can be provided based on the determination result, to complete the present invention.

That is, the present invention includes the following inventive aspects.
(1) A chewing assistance system including an information processing device that includes: chewing information storage means that stores information about chewing quality; moving image obtaining means that obtains a moving image of a region including at least a mouth or a peripheral portion of the mouth in a face; analysis means that analyzes a chewing action based on the moving image of the region obtained by the moving image obtaining means; quality determination means that determines quality of the chewing action based on information of the chewing action analyzed by the analysis means; and extraction means that extracts assistance information corresponding to the chewing quality determined by the quality determination means, from the chewing information storage means.
(2) In the chewing assistance system according to the above-described (1), the analysis means includes feature detection means that detects a feature point in a face from an image of the region, and action analysis means that analyzes an action based on change of the feature point detected by the feature detection means.
(3) In the chewing assistance system according to the above-described (2), the action analysis means determines, in a case where a quantity of change of the feature point indicates a value that exceeds a predetermined threshold value, that the change is caused by chewing, and analyzes the action of the chewing.
(4) In the chewing assistance system according to the above-described (2) or (3), the feature point includes at least one of a nasal tip, a nasion, a corner of a mouth, a vertex of an upper lip, a vertex of a lower lip, a vertex of a jaw, and a point along an outline of a cheek near masseter.
(5) In the chewing assistance system according to any one of the above-described (2) to (4), the change of the feature point includes at least one of change of a position of the feature point, change of a distance between two feature points, and change of an area surrounded by three or more feature points.
(6) In the chewing assistance system according to any one of the above-described (1) to (5), the chewing action analyzed by the analysis means includes an action associated with at least one of a total number of chewing times, chewing rhythm, a motion of a mouth, a motion of a jaw, occlusal balance between anterior and posterior sides/between left and right sides, and a motion of masseter.
(7) In the chewing assistance system according to any one of the above-described (1) to (6), the quality of the chewing action determined by the quality determination means includes quality based on at least one of determinations as to whether a total number of chewing times is large or small, whether chewing rhythm is proper, whether mouth opening behavior is proper, whether chewing balance between a left side and a right side is proper, whether eating behavior (motion of a mouth) is proper, and whether use of masseter is proper.
(8) In the chewing assistance system according to any one of the above-described (1) to (7), the quality determination means compares a chewing action with a previous chewing action of a same person and determines whether the chewing action has improved.
(9) In the chewing assistance system according to any one of the above-described (1) to (8), the quality determination means has a machine learning mechanism, and the quality of the chewing action is determined with reference to a learning result from the machine learning mechanism.
(10) A chewing assistance program including a control program for causing an information processing device to function as the chewing assistance system according to any one of claims 1 to 9, the chewing assistance program causing the information processing device to function as the moving image obtaining means, the analysis means, the quality determination means, and the extraction means.

### ADVANTAGEOUS EFFECTS OF THE INVENTION

According to the present invention described above, a chewing action is analyzed from a moving image of a region including at least a mouth or a peripheral portion of the mouth in a face so as to determine quality of the chewing action, and assistance information corresponding to the determined chewing quality can be provided. Therefore, chewing quality during a meal can be obtained in a simple method without attaching a device such as an electromyograph to the body and causing a user to feel uncomfortable and applying a load on the user, and improvement of chewing quality can be supported.

The present invention having such a configuration can provide the system that can provide growth information about quality of healthy chewing, in particular, for growing children in a simple manner, and that contributes to healthy development of a chewing action function for the growing children. Also for elderly people, the present invention can provide the system that can provide assistance information corresponding to chewing quality in a simple manner before, for example, a device is attached and measurement is performed, and that contributes to maintenance and enhancement of a chewing action function of elderly people.

### BRIEF DESCRIPTION OF THE DRAWINGS

[FIG. 1] FIG. 1 is a block diagram illustrating a configuration of a chewing assistance system according to a representative embodiment of the present invention.
[FIG. 2] FIG. 2 is an explanatory diagram that illustrates feature points, in a face, detected by a feature detection processing unit of the chewing assistance system described above.
[FIG. 3A] FIG. 3A illustrates a graph obtained when a feature quantity calculation processing unit of the chewing assistance system described above calculates, as a feature quantity, a relative position coordinate of a vertex of an upper lip relative to a nasal tip.
[FIG. 3B] FIG. 3B illustrates a graph obtained by calculating, as the feature quantity, a distance from the nasal tip to a vertex of an upper lip.
[FIG. 4] FIG. 4 is an explanatory diagram that illustrates a method, performed by an action analysis processing unit of the chewing assistance system described above, for performing rectification by using a low-cut filter and determining, as chewing, motion that indicates values greater than certain threshold values (±x), based on the graph illustrated in FIG. 3A.
[FIG. 5A] FIG. 5A is an explanatory diagram that illustrates a state where lines extending across 0 are extracted as one chewing one by one.
[FIG. 5B] FIG. 5B is an explanatory diagram that illustrates a state where the V shape is determined as one chewing action and a motion in the chewing action is extracted.
[FIG. 6A] FIG. 6A illustrates a graph obtained when the feature quantity calculation processing unit of the chewing assistance system described above calculates, as the feature quantity, a relative position coordinate of a vertex of a lower lip relative to a nasal tip.
[FIG. 6B] FIG. 6B illustrates a graph obtained by calculating, as the feature quantity, a distance from a nasal tip to a vertex of a lower lip.
[FIG. 7A] FIG. 7A illustrates a graph obtained when the feature quantity calculation processing unit of the chewing assistance system described above calculates, as the feature quantity, a relative position coordinate of a right mouth corner relative to a nasal tip.
[FIG. 7B] FIG. 7B illustrates a graph obtained by calculating, as the feature quantity, a distance from a nasal tip to a right mouth corner.
[FIG. 8A] FIG. 8A illustrates a graph obtained when the feature quantity calculation processing unit of the chewing assistance system described above calculates, as the feature quantity, a relative position coordinate of a left mouth corner relative to a nasal tip.
[FIG. 8B] FIG. 8B illustrates a graph obtained by calculating, as the feature quantity, a distance from a nasal tip to a left mouth corner.
[FIG. 9A] FIG. 9A illustrates a graph obtained when the feature quantity calculation processing unit of the chewing assistance system described above calculates, as the feature quantity, a relative position coordinate of a vertex of a jaw relative to a nasal tip.
[FIG. 9B] FIG. 9B illustrates a graph obtained by calculating, as the feature quantity, a distance from a nasal tip to a vertex of a jaw.
[FIG. 10A] FIG. 10A illustrates a graph obtained when the feature quantity calculation processing unit of the chewing assistance system described above calculates, as the feature quantity, a relative position coordinate of a right mouth corner relative to a left mouth corner.
[FIG. 10B] FIG. 10B illustrates a graph obtained by calculating, as the feature quantity, a distance from a left mouth corner to a right mouth corner.
[FIG. 11A] FIG. 11A illustrates a graph obtained when the feature quantity calculation processing unit of the chewing assistance system described above calculates, as the feature quantity, a relative position coordinate of a vertex of a lower lip relative to a vertex of an upper lip.
[FIG. 11B] FIG. 11B illustrates a graph obtained by calculating, as the feature quantity, a distance from a vertex of an upper lip to a vertex of a lower lip.
[FIG. 12A] FIG. 12A is an explanatory diagram that illustrates a state where the feature quantity calculation processing unit of the chewing assistance system described above calculates, as the feature quantity, a left-side area and a right-side area which correspond to masseter and which are surrounded by mouth corners and a plurality of predetermined positions (feature points) along an outline of a cheek.
[FIG. 12B] FIG. 12B is an explanatory diagram that illustrates a state where the feature quantity calculation processing unit of the chewing assistance system described above calculates, as the feature quantity, a left-side area and a right-side area which correspond to masseter and which are surrounded by mouth corners and a plurality of predetermined positions (feature points) along an outline of a cheek.
[FIG. 12C] FIG. 12C is an explanatory diagram that illustrates a state where the feature quantity calculation processing unit of the chewing assistance system described above calculates, as the feature quantity, a left-side area and a right-side area which correspond to masseter and which are surrounded by mouth corners and a plurality of predetermined positions (feature points) along an outline of a cheek.
[FIG. 13A] FIG. 13A illustrates a graph obtained when the feature quantity calculation processing unit described above calculates, as the feature quantity, a left-side area and a right-side area which correspond to masseter and which are surrounded by mouth corners and a plurality of predetermined positions (feature points) along an outline of a cheek.
[FIG. 13B] FIG. 13B illustrates a graph obtained when the feature quantity calculation processing unit described above calculates, as the feature quantity, a left-side area and a right-side area which correspond to masseter and which are surrounded by mouth corners and a plurality of predetermined positions (feature points) along an outline of a cheek.
[FIG. 13C] FIG. 13C illustrates a graph obtained when the feature quantity calculation processing unit described above calculates, as the feature quantity, a left-side area and a right-side area which correspond to masseter and which are surrounded by mouth corners and a plurality of predetermined positions (feature points) along an outline of a cheek.
[FIG. 14A] FIG. 14A is an explanatory diagram that illustrates a pattern (trajectory) of the feature points in one chewing section in the case of chewing being performed on a left side.
[FIG. 14B] FIG. 14B similarly illustrates a pattern (trajectory) of the feature points in one chewing section in the case of chewing being performed on a left side.
[FIG. 14C] FIG. 14C similarly illustrates a pattern (trajectory) of the feature points in one chewing section in the case of chewing being performed on the left side.
[FIG. 14D] FIG. 14D similarly illustrates a pattern (trajectory) of the feature points in one chewing section in the case of chewing being performed on the left side.
[FIG. 15A] FIG. 15A illustrates a pattern (trajectory) of the feature points in one chewing section in the case of chewing being performed on a right side.
[FIG. 15B] FIG. 15B similarly illustrates a pattern (trajectory) of the feature points in one chewing section in the case of chewing being performed on the right side.
[FIG. 15C] FIG. 15C similarly illustrates a pattern (trajectory) of the feature points in one chewing section in the case of chewing being performed on the right side.
[FIG. 15D] FIG. 15D similarly illustrates a pattern (trajectory) of the feature points in one chewing section in the case of chewing being performed on the right side.
[FIG. 16] FIG. 16 is a flowchart showing a procedure of processing performed by the chewing assistance system according to the representative embodiment.
[FIG. 17A] FIG. 17A illustrates motion of a jaw in the case of chewing being performed on the right side.
[FIG. 17B] FIG. 17B illustrates motion of the jaw in the case of chewing being performed on the right side.

### DESCRIPTION OF EMBODIMENTS

Next, an embodiment of the present invention will be described in detail with reference to the accompanied drawings.

Chewing during a meal is associated with a person's favorite hardness/softness of food, a motion of biting through and masticating the food, the number of times of chewing the food, a chewing time, rhythm, and the like. Balance between chewing teeth is also among them. For determining chewing quality based on whether or not such a function of chewing and eating deliciously is proper, the system of the present invention measures, as chewing quality, the number of chewing times, chewing rhythm, eating behavior (grinding? moving only in the up-downward direction? lips remain opened?), a motion of the jaw during chewing, an occlusal balance (among the anterior, the posterior, the left, and the right sides), a motion of muscle, and the like by analyzing moving images, to indicate the chewing quality as, for example, a numerical value, and further indicates change with the elapse of time according to difference between the past state and the present state, for example. Therefore, the system of the present invention can present an improving state of the chewing quality.

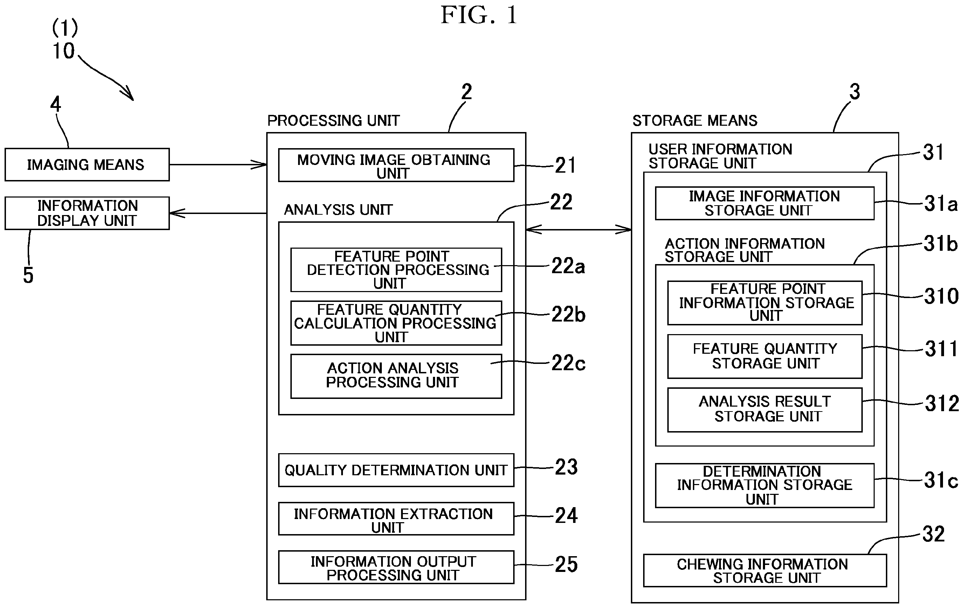

Specifically, as illustrated in FIG. 1, a chewing assistance system 1 of the present invention is configured by a single or a plurality of information processing devices 10 each including a processing unit 2, storage means 3, imaging means 4, and an information display unit 5. Specifically, the information processing device 10 is implemented by, for example, a computer that includes the processing unit 2 as a main unit and also includes, for example, storage means, input means such as a pointing device, a keyboard, and a touch panel, display means such as a display, and another unit such as a communication controller which is not illustrated.

The processing unit 2 includes a CPU such as a microprocessor as a main unit and also has a not-illustrated storage unit, such as a RAM and a ROM, in which a program for providing procedures of various processing operations, and process data are stored. The storage means 3 includes a memory, a hard disk, and the like disposed inside and/or outside the information processing device 10. A part or all of contents in the storage unit may be stored in, for example, a hard disk or a memory of another computer that is connected to the information processing device 10 so as to be communicable with each other. The information processing device having such a configuration may be a dedicated device that is installed in a dental clinic, a hospital, another institution, a store, or the like, or may be a general-purpose household personal computer. The information processing device may be, for example, a smartphone carried by a user.

The processing unit 2 incudes, as its functions, a moving image obtaining unit 21 as moving image obtaining means, an analysis unit 22, a quality determination unit 23 as quality determination means, an information extraction unit 24, and an information output processing unit 25. The moving image obtaining unit 21 obtains two-dimensional or three-dimensional moving image information, of a region including at least a mouth or a peripheral portion of the mouth in a face of a user, which is obtained and transmitted by the imaging means 4, and stores the moving image information in an image information storage unit 31a of a user information storage unit 31. The analysis unit 22 analyzes a chewing action based on the moving image information, and stores information of the analyzed chewing action in an action information storage unit 31b of the user information storage unit 31. The quality determination unit 23 determines quality of the chewing action based on the information of the chewing action, and stores information of the determined quality of the chewing action in a determination information storage unit 31c of the user information storage unit 31. The information extraction unit 24 receives input of the information of the determined chewing quality, and extracts information to be recommended from information, about the chewing quality, stored in a chewing information storage unit 32. The information output processing unit 25 presents the information to the user by, for example, displaying the information on a display (information display unit 5). These processing functions are executed by the above-described program.

The imaging means 4 is implemented by a CCD camera or the like, and may be a CCD camera included in a smartphone of a user configured as the information processing device 10 or may also be implemented by, for example, an external camera connected to, for example, a dedicated computer device as the information processing device 10. 3D imaging or the like can be utilized. The imaging means 4 obtains moving image information of a region including at least a mouth or a peripheral portion of the mouth in a face of a user, preferably, a region including a nasal tip, a nasion, a corner of a mouth, a vertex of an upper lip, a vertex of a lower lip, a vertex of a jaw, and a cheek near masseter, each of which serves as "feature point" described below.

The analysis unit 22 functions as analysis means, and more specifically includes a feature detection processing unit 22a, a feature quantity calculation processing unit 22b, and an action analysis processing unit 22c. The feature detection processing unit 22a detects feature points in a face from the image of the above-described region, and stores position information thereof in a feature point information storage unit 310 of the action information storage unit 31b. The feature quantity calculation processing unit 22b calculates a feature quantity to be used for analyzing an action, based on the positions of the detected feature points, and stores the feature quantity in a feature quantity storage unit 311. The action analysis processing unit 22c analyzes an action based on change of the calculated feature quantity.

The feature detection processing unit 22a can use, as a technique for detecting a face feature point, various known methods such as an active shape model (ASM), an active appearance model (AAM), and a constrained local model (CLM). As the feature points to be detected in a face, a nasal tip, a nasion, a corner of a mouth, a vertex of an upper lip, a vertex of a lower lip, a vertex of a jaw, and a point along an outline of a cheek near masseter are preferably used.

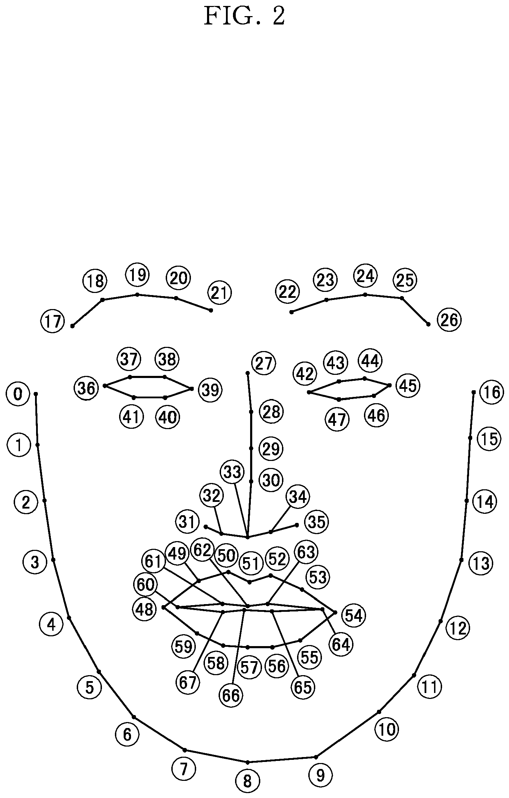

FIG. 2 illustrates examples of the detected feature points in a face. In this example, 68 feature points from points "0" to "67" are detected. The feature detection processing unit 22a stores the detected feature points in the face, together with position information thereof, in the feature point information storage unit 310.

Preferable examples of the feature quantity calculated by the feature quantity calculation processing unit 22b include relative positions and distances of points such as left and right mouth corners (feature points), a vertex (feature point) of an upper lip, a vertex (feature point) of a lower lip, and a vertex (feature point) of a jaw, relative to a nasal tip (feature point) or a nasion (feature point) which is not affected during chewing, and a distance as a thickness of a lip between a vertex (feature point) of an upper lip and a vertex (feature point) of a lower lip, a distance as a width of a lip between left and right mouth corners (feature points), and areas which correspond to masseter and are surrounded by mouth corners (feature points) and a plurality of predetermined positions (feature points) along an outline of a cheek.

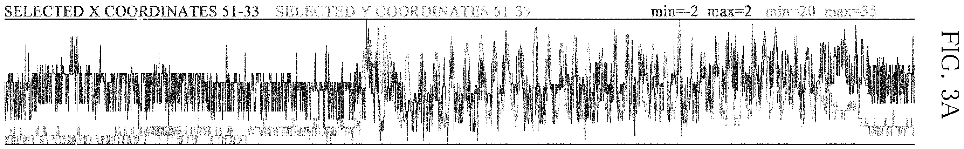

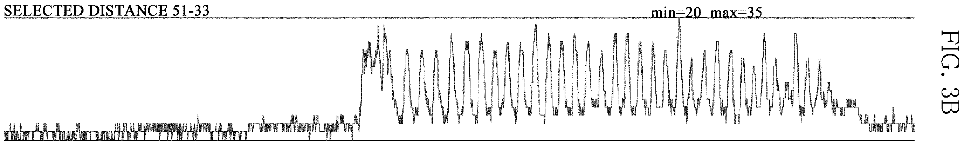

FIG. 3A illustrates a graph obtained when the feature quantity calculation processing unit 22b calculates, as the feature quantity, a relative position coordinate of a vertex "51" of an upper lip relative to a nasal tip "33" among the detected feature points in a face as illustrated in FIG. 2. FIG. 3B illustrates a graph obtained when the feature quantity calculation processing unit 22b calculates, as the feature quantity, a distance from the nasal tip "33" to the vertex "51" of the upper lip. The horizontal axis represents time and the vertical axis represents feature quantity.

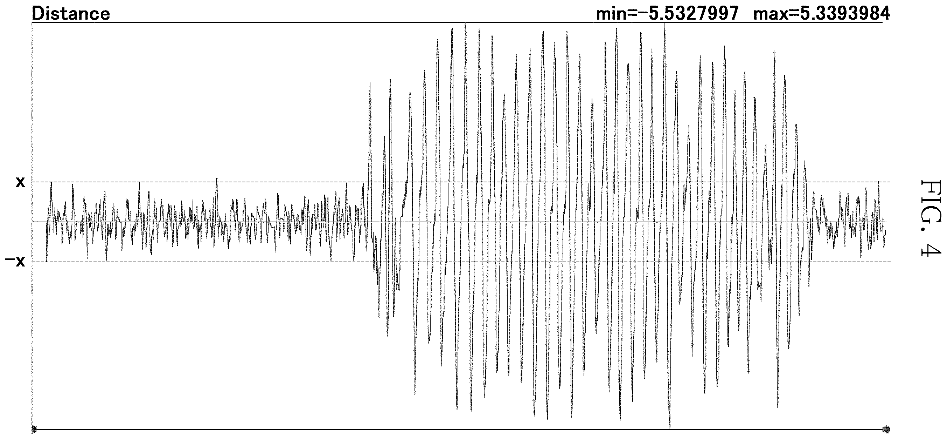

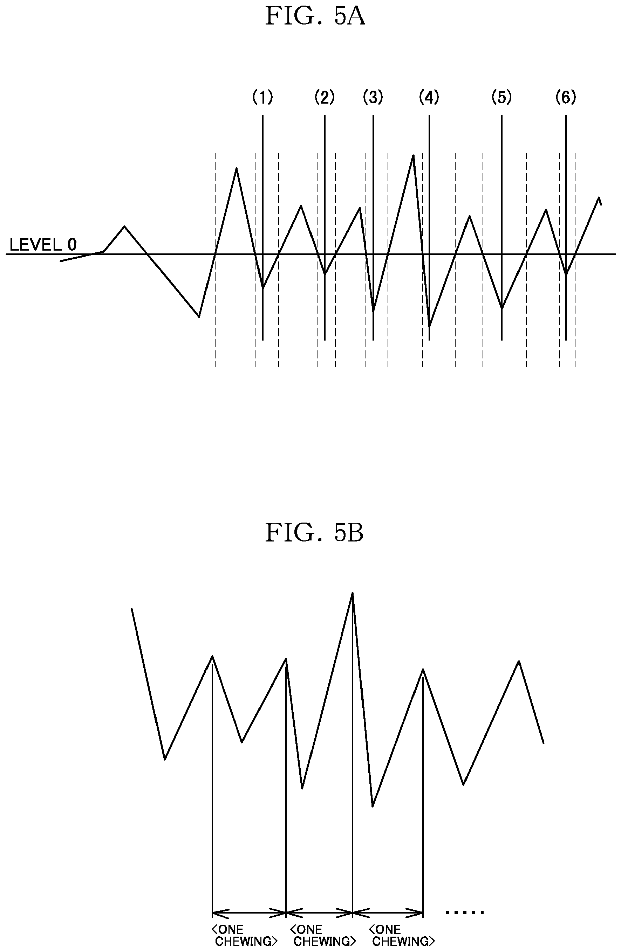

Based on such a graph representing the calculated change of the distance (feature quantity) from the nasal tip "33" to the vertex "51" of the upper lip as illustrated in FIG. 3B, for example, the action analysis processing unit 22c performs rectification by using a low-cut filter as illustrated in FIG. 4, and then determines, as chewing, motion indicating values greater than certain threshold values (±x), extracts one chewing (chewing (1), chewing (2), chewing (3), ...) one by one as illustrated in FIG. 5A, and thus determines a chewing action (the number of chewing times, chewing rhythm, and the like). In a case where a value that does not exceed the threshold value is included in one chewing, a chewing action may be determined so as to exclude this chewing.

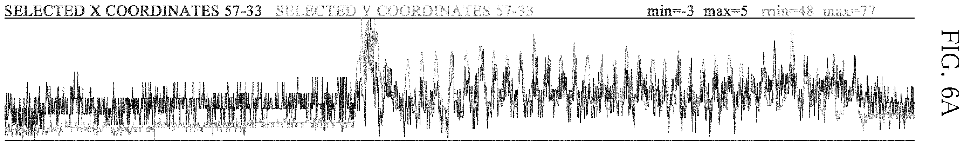

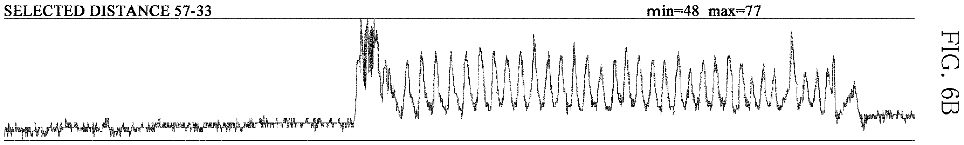

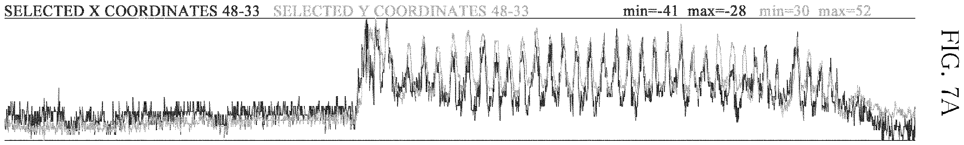

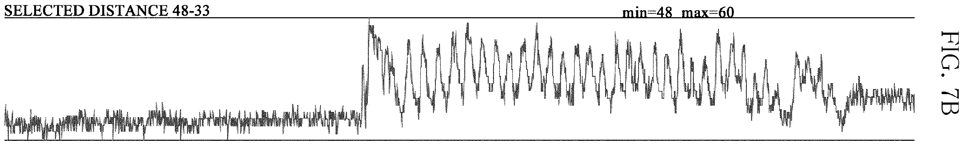

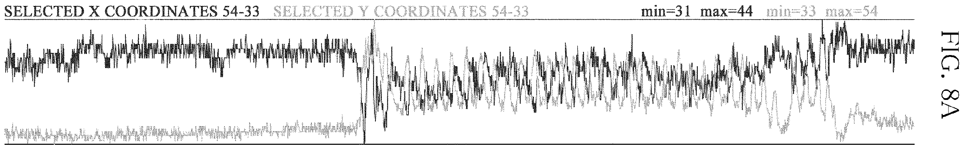

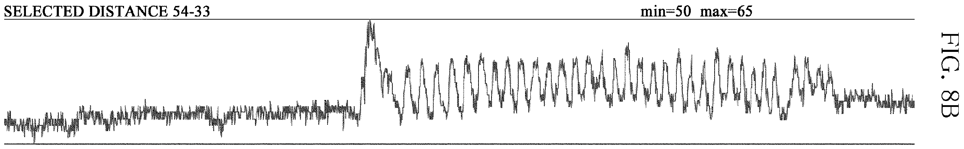

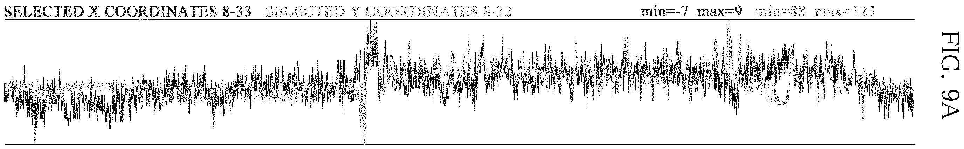

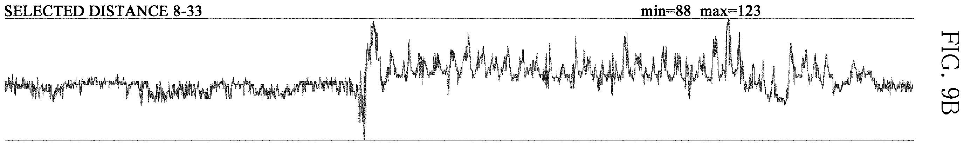

Examples in which the feature quantity calculation processing unit 22b similarly calculates the feature quantity are as follows. For example, a relative position coordinate of a vertex "57" of a lower lip relative to the nasal tip "33" and a distance therebetween are each calculated as the feature quantity as illustrated in FIG. 6A and FIG. 6B, a relative position coordinate of a right mouth corner "48" relative to the nasal tip "33" and a distance therebetween are each calculated as the feature quantity as illustrated in FIG. 7A and FIG. 7B, a relative position coordinate of a left mouth corner "54" relative to the nasal tip "33" and a distance therebetween are each calculated as the feature quantity as illustrated in FIG. 8A and FIG. 8B, and a relative position coordinate of a vertex "8" of a jaw relative to the nasal tip "33" and a distance therebetween are each calculated as the feature quantity as illustrated in FIG. 9A and FIG. 9B. The action analysis processing unit 22c can determine the chewing action based on the graphs of the distances as described above.

Thus, the chewing action, specifically, the number of chewing times, rhythm, chewing balance between the left side and the right side, and the like can be analyzed by analyzing a relative position and a distance of each of points such as left and right mouth corners, a vertex of an upper lip, a vertex of a lower lip, and a vertex of a jaw relative to a nasal tip or a nasion which is not affected during chewing.

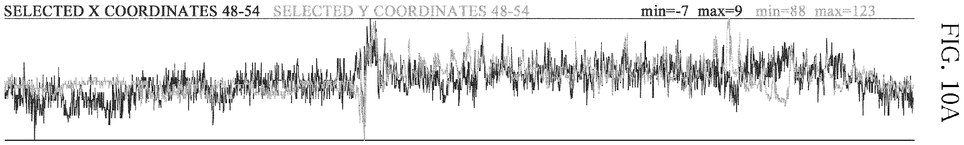

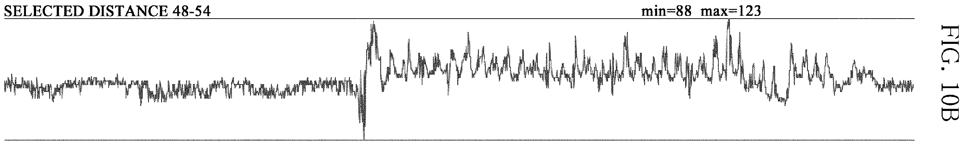

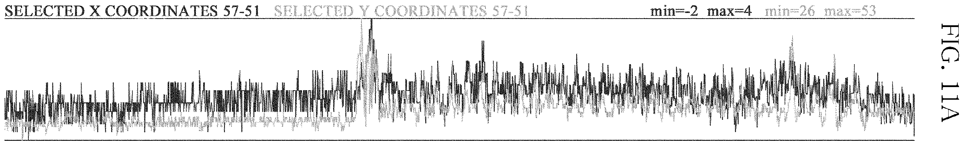

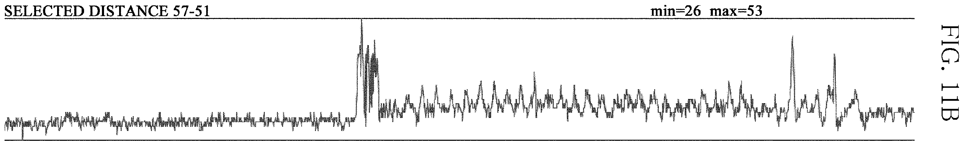

Furthermore, examples in which the feature quantity calculation processing unit 22b similarly calculates the feature quantity are as follows A relative position coordinate of the right mouth corner "48" relative to the left mouth corner "54" and a distance therebetween are each calculated as the feature quantity as illustrated in FIG. 10A and FIG. 10B, and a relative position coordinate of the vertex "57" of the lower lip relative to the vertex "51" of the upper lip and a distance therebetween are each calculated as the feature quantity as illustrated in FIG. 11A and FIG. 11B. In a case where the relative position coordinate and the distance between the left mouth corner and the right mouth corner, and the relative position coordinate and the distance between the vertex of the upper lip and the vertex of the lower lip are calculated as the feature quantities as described above, the action analysis processing unit 22c can more directly analyze mouth opening behavior during chewing, for example, analyze motion of the mouth which represents a size or a shape of the opened mouth, or the like.

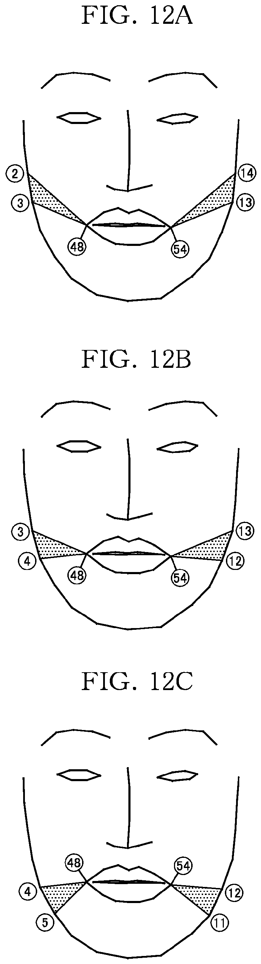

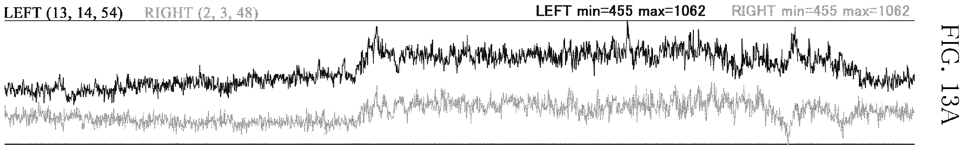

Other examples in which the feature quantity calculation processing unit 22b calculates the feature quantity are as follows. A left-side area and a right-side area which correspond to masseter and which are surrounded by mouth corners and a plurality of predetermined positions (feature points) along an outline of a cheek are calculated as the feature quantities as illustrated in FIG. 12A to FIG. 12C and FIG. 13A to FIG. 13C. In the examples in FIG. 12A to FIG. 12C and FIG. 13A to FIG. 13C, the left-side area and the right-side area are each divided into three regions that are an upper region, a mid-region, and a lower region.

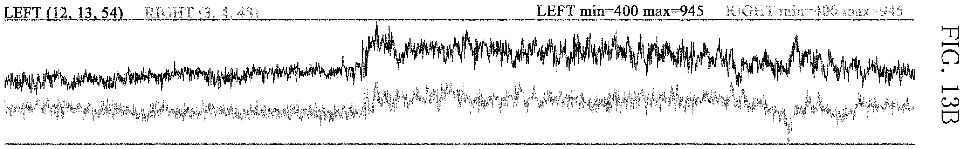

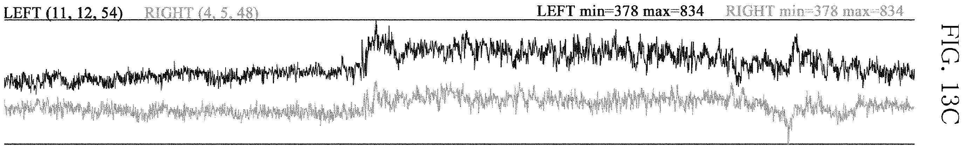

In the upper region illustrated in FIG. 12A and FIG. 13A, an area of a triangular shape having "2", "3", and "48" as vertexes is a right-side region, and an area of a triangular shape having "13", "14", and "54" as vertexes is a right-side region. Similarly, in the mid-region illustrated in FIG. 12B and FIG. 13B, an area of a triangular shape having "3", "4", and "48" as vertexes is a right-side region, and an area of a triangular shape having "12", "13", and "54" as vertexes is a right-side region. Similarly, in the lower region illustrated in FIG. 12C and FIG. 13C, an area of a triangular shape having "4", "5", and "48" as vertexes is a right-side region and an area of a triangular shape having "11", "12", and "54" as vertexes is a right-side region.

Thus, in a case where a left-side area and a right-side area surrounded by mouth corners and a plurality of predetermined positions along an outline of a cheek are calculated as the feature quantities, the action analysis processing unit 22c can more directly analyze, for example, a way of exerting a force in a mouth during chewing, i.e., motion of masseter, and chewing balance, caused by the motion, between the anterior and posterior sides/between the left and right sides.

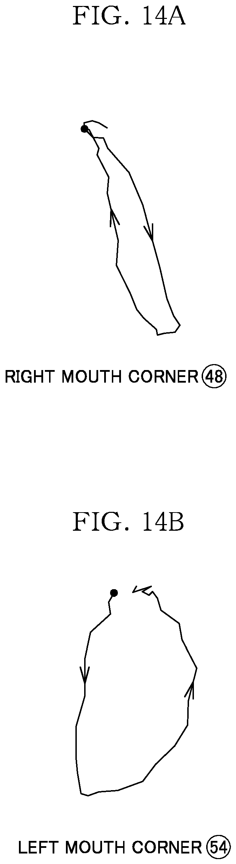

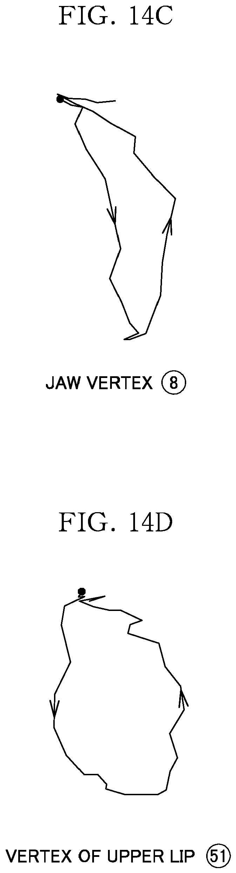

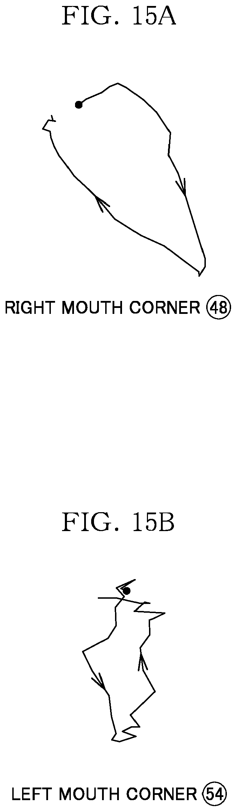

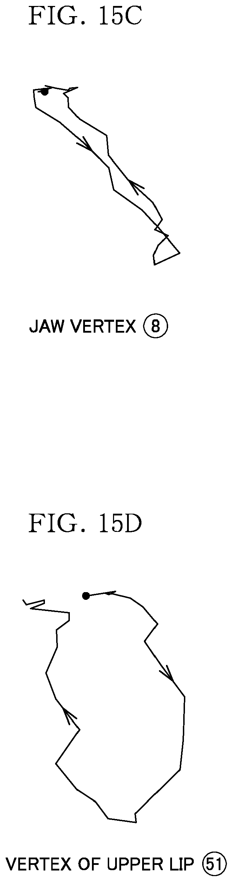

Furthermore, the motions of the respective feature points are defined as patterns based on position coordinates, of mouth corners, the jaw, the vertexes of the upper lip and the lower lip, and the like, calculated by the feature quantity calculation processing unit 22b, and pattern determination may be performed by a machine learning mechanism or the like to analyze a chewing action. The motion of each feature point is preferably extracted such that, for example, the V shape is determined as one chewing action and a motion in the chewing action is extracted as illustrated in FIG. 5B. Specifically, FIG. 14A to FIG. 14D illustrate patterns (trajectories) of the feature points (the right mouth corner "48", the left mouth corner "54", the jaw vertex "8", the vertex "51" of the upper lip) in one chewing section in the case of chewing being performed on the left side. FIG. 15A to FIG. 15D illustrate patterns (trajectories) of the feature points (the right mouth corner "48", the left mouth corner "54", the jaw vertex "8", the vertex "51" of the upper lip) in one chewing section in the case of chewing being performed on the right side.

Thus, by defining, as a pattern, a trajectory of each of the feature points during chewing, motion (mouth opening behavior, chewing balance between the left side and the right side, and the like) of a mouth during chewing can be more accurately analyzed. Preferably, the action analysis processing unit 22c has such a machine learning mechanism, and determines each of the above-described actions with reference to learning results from the machine learning mechanism.

The quality of the chewing action determined by the quality determination unit 23 includes, for example, quality based on determination as to whether the number of chewing times is large or small, whether or not chewing rhythm is proper, whether or not mouth opening behavior is proper, whether or not chewing balance between the left side and the right side is proper, whether or not eating behavior (motion of a mouth) is proper, and whether or not use of masseter is proper.

For example, information as to whether or not a user has improved chewing quality as compared with the her/his previous quality and information as to whether or not the chewing quality is commensurate with the age are preferably obtained so as to be included in the quality, according to the obtained data, previous information for the user in the determination information storage unit 31c, and age-based statistical information. Preferably, the quality determination unit 23 has a machine learning mechanism, and determines the quality of the chewing action with reference to learning results from the machine learning mechanism.

The information extraction unit 24 functions as extraction means. For example, if the chewing quality is not commensurate with the age, the information extraction unit 24 preferably extracts information such as age-based oral cavity function information, and information about a device for growing/improving purpose and a medical specialist based on a residence of the user.

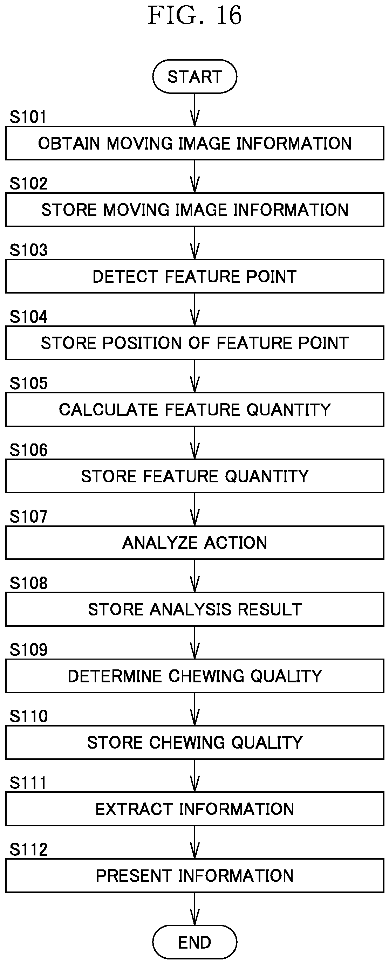

FIG. 16 is a flowchart showing a procedure of processing performed by the chewing assistance system 1 of the present embodiment.

Firstly, the moving image obtaining unit 21 obtains, from the imaging means 4, moving image information of a face of a user at least from putting of prescribed food (predetermined food) or ordinary food in a mouth up to swallowing (S101), and stores the moving image information in the image information storage unit 31a of the user information storage unit 31 (S102).

Subsequently, the feature detection processing unit 22a detects feature points in the face from the image of the region (S103), and stores position information of the feature points in the feature point information storage unit 310 of the action information storage unit 31b (S104). The feature quantity calculation processing unit 22b calculates the feature quantity to be used for analyzing an action based on the positions of the detected feature points (S105) and stores the feature quantity in the feature quantity storage unit 311 (S106).

Subsequently, the action analysis processing unit 22c analyzes an action based on change of the calculated feature quantity (S107), and stores an analysis result in the analysis result storage unit 312 (S108). Subsequently, the quality determination unit 23 determines quality of the chewing action based on the analysis result (S109), and stores information of the determined quality of the chewing action in the determination information storage unit 31c of the user information storage unit 31 (S110).

Subsequently, the information extraction unit 24 receives input of the information of the determined chewing quality and extracts information to be recommended from information of chewing quality stored in the chewing information storage unit 32 (S111). The information output processing unit 25 presents the extracted information to the user by, for example, displaying the information on a display (the information display unit 5) (S112).

Although the embodiment of the present invention has been described above, the present invention is not limited to the embodiment at all. For example, instead of the processing unit being configured by software processing performed by a computer, it is also preferable that a part or the entirety of the processing unit is configured by a hardware processing circuit. In this case, a processing circuit for artificial intelligence can also be used as the machine learning mechanism, and it is needless to say that the present invention can be implemented in various modes without departing from the gist of the present invention.

### INDUSTRIAL APPLICABILITY

According to the present invention, chewing quality is determined in simple means in which a moving image of a mouth or a peripheral portion of the mouth is taken, and assistance information based on the determination result can be provided. Therefore, by combining the present invention with instruments and commodities/services for education and training of chewing for children, commodities and services contributing to healthy development of children can be provided. Furthermore, by combining the present invention with cosmetic training instruments and services for, for example, use of masticatory muscle and well-balanced chewing among the anterior, the posterior, the left, and the right sides, cosmetic commodities and services for preventing distortion of the face and obesity, and maintaining vital healthy facial expression can also be provided. Moreover, by combining the present invention with commodities and services for addressing oral frailty such as deterioration of an oral cavity function and weakening of the body for elderly people and the like, commodities and services contributing to extension of healthy life expectancy can also be provided.

### DESCRIPTION OF THE REFERENCE CHARACTERS

- 1: chewing assistance system
- 2: processing unit
- 3: storage means
- 4: imaging means
- 5: information display unit
- 10: information processing device
- 21: moving image obtaining unit
- 22: analysis unit
- 22a: feature detection processing unit
- 22b: feature quantity calculation processing unit
- 22c: action analysis processing unit
- 23: quality determination unit
- 24: information extraction unit
- 25: information output processing unit
- 31: user information storage unit
- 31a: image information storage unit
- 31b: action information storage unit
- 31c: determination information storage unit
- 32: chewing information storage unit
- 310: feature point information storage unit
- 311: feature quantity storage unit
- 312: analysis result storage unit

## Claims

1. A chewing assistance system comprising an information processing device that includes:
chewing information storage means that stores information about chewing quality;
moving image obtaining means that obtains a moving image of a region including at least a mouth or a peripheral portion of the mouth in a face;
analysis means that analyzes a chewing action based on the moving image of the region obtained by the moving image obtaining means;
quality determination means that determines quality of the chewing action based on information of the chewing action analyzed by the analysis means; and
extraction means that extracts assistance information corresponding to the chewing quality determined by the quality determination means, from the chewing information storage means.

2. The chewing assistance system according to claim 1, wherein
the analysis means includes
feature detection means that detects a feature point in a face from an image of the region, and
action analysis means that analyzes an action based on change of the feature point detected by the feature detection means.

3. The chewing assistance system according to claim 2, wherein
the action analysis means
determines, in a case where a quantity of change of the feature point indicates a value that exceeds a predetermined threshold value, that the change is caused by chewing, and analyzes the action of the chewing.

4. The chewing assistance system according to claim 2 or 3, wherein
the feature point
includes at least one of a nasal tip, a nasion, a corner of a mouth, a vertex of an upper lip, a vertex of a lower lip, a vertex of a jaw, and a point along an outline of a cheek near masseter.

5. The chewing assistance system according to any one of claims 2 to 4, wherein
the change of the feature point includes at least one of change of a position of the feature point, change of a distance between two feature points, and change of an area surrounded by three or more feature points.

6. The chewing assistance system according to any one of claims 1 to 5, wherein
the chewing action analyzed by the analysis means
includes an action associated with at least one of a total number of chewing times, chewing rhythm, a motion of a mouth, a motion of a jaw, occlusal balance between anterior and posterior sides/between left and right sides, and a motion of masseter.

7. The chewing assistance system according to any one of claims 1 to 6, wherein
the quality of the chewing action determined by the quality determination means includes quality based on at least one of determinations as to whether a total number of chewing times is large or small, whether chewing rhythm is proper, whether mouth opening behavior is proper, whether chewing balance between a left side and a right side is proper, whether eating behavior (motion of a mouth) is proper, and whether use of masseter is proper.

8. The chewing assistance system according to any one of claims 1 to 7, wherein
the quality determination means
compares a chewing action with a previous chewing action of a same person and determines whether the chewing action has improved.

9. The chewing assistance system according to any one of claims 1 to 8, wherein
the quality determination means has a machine learning mechanism, and
the quality of the chewing action is determined with reference to a learning result from the machine learning mechanism.

10. A chewing assistance program comprising a control program for causing an information processing device to function as the chewing assistance system according to any one of claims 1 to 9, the chewing assistance program causing the information processing device to function as the moving image obtaining means, the analysis means, the quality determination means, and the extraction means.
